# EUROPEAN PATENT SPECIFICATION

(11) **EP 4 240 479 B1**
(45) Date of publication and mention of the grant of the patent: **18.02.2026**
(21) Application number: 21811264.7
(22) Date of filing: 08.11.2021
(51) Int. Cl.: A61N 2/02, A61N 2/12, A61N 2/00

(54) **SYSTEM FOR STIMULATION OF BIOLOGICAL TISSUE**
SYSTEM ZUR STIMULATION VOM BIOLOGISCHEN GEWEBE
SYSTÈME DE STIMULATION DES TISSUS BIOLOGIQUES

(30) Priority: 09.11.2020 EP 20206444
(43) Date of publication of application: 13.09.2023
(73) Proprietor: Charité Universitätsmedizin Berlin, 10117 Berlin (DE)
(72) Inventor: SOEKADAR, Surjo Raphael, 12249 Berlin (DE); NASR, Khaled, 10557 Berlin (DE)
(74) Representative: Maikowski & Ninnemann Patentanwälte Partnerschaft mbB
(86) International application number: PCT/EP2021/080940
(87) International publication number: WO 2022/096710

(56) References cited:
- WO-A1-2016/134367
- WO-A1-2017/002065
- WO-A1-2018/185369
- WO-A2-00/78267
- WO-A2-2010/100643
- RU-C1- 2 012 387
- RU-C2- 2 228 208
- US-A- 5 480 373
- US-A- 5 562 706
- US-B1- 6 461 289

## Description

The invention relates to a system for stimulation of biological tissue according to claim 1 and to a computer program product according to claim 15.

Non-invasive brain stimulation (NIBS) devices are valuable tools that can potentially be used to modulate neural activity and investigate the causal role that different brain regions and oscillations play in behavior, or to treat various psychiatric disorders. The two most widely-applied NIBS techniques are transcranial magnetic stimulation (TMS) and transcranial electric stimulation (TES). In TMS, an electromagnetic coil is placed on the scalp and e.g. driven with high-current pulses. The pulses induce a strong time-varying magnetic field in the vicinity of the coil which in turn induces an electric field in the brain regions under the coil. Furthermore, the article by M. Zaeimbashi et al., "Magnetic Temporal Interference for Noninvasive, High-resolution, and Localized Deep Brain Stimulation: Concept Validation", bioRxiv, doi: https://doi.org/10.1101/2020.07.20.212845 describes using interfering high frequency magnetic fields of slightly different frequencies, wherein peripheral regions of the brain are affected by the high frequency magnetic fields, while in the interference region a magnetic field having a low frequency envelope is formed which can stimulate the corresponding brain region. However, the shape of the envelope is unchangeable such that the stimulation pattern is restricted.

Another magnetic stimulation device is described in publication RU 2 228 208 C2.

It is an object of the invention to provide a system permitting a less restricted stimulation pattern. The invention is defined by the claims.

According to invention, a system for (in particular magnetic) stimulation of biological tissue is provided, comprising
- at least two magnetic field generating devices for generating time-varying magnetic fields, wherein the two magnetic field generating devices are to be arranged relative to the tissue in such a way that their time-varying magnetic fields and/or electric fields induced by the time-varying magnetic fields at least partially overlap in the tissue;
- at least one controlling arrangement for controlling the two magnetic field generating devices, wherein
- the controlling arrangement is configured to control at least one of the two magnetic field generating devices in such a way that the time-varying magnetic field generated by the at least one device is modulated (time modulated, more particularly phase modulated).

The time-varying magnetic fields generated by the magnetic field generating devices and emitted into the biological tissue (e.g. neural tissue) and/or the electric fields induced in the tissue by the magnetic fields will interfere with one another in the region of the tissue where the fields overlap. The resulting magnetic and/or induced electric field in the overlap region will be amplitude-modulated at a frequency that equals or at least depends on the modulation frequency. The frequency of the amplitude modulation in any case will be lower than the frequency of the time-varying magnetic fields emitted into the tissue such that the tissue (in particular neural tissue) may not respond to the time-varying magnetic and/or induced electric fields, but only to the amplitude-modulated resulting fields. The effect on the tissue thus may be confined to the overlap region of the time-varying magnetic and/or induced electric fields. For example, the frequency of the modulation is lower than a third or a fifth of the frequency of the time-varying magnetic field (i.e. the carrier frequency). The carrier frequency of the time-varying magnetic fields may be in the kHz range (e.g. at least 1 KHz), while the modulation frequency may be below 500 Hz, below 300 or below 200 Hz. For example, the carrier frequency of at least one of the time-varying magnetic fields (e.g. of the one that is phase modulated) is at least 1 kHz. Further, the carrier frequency of at least one of the time-varying magnetic fields (e.g. the one whose carrier frequency is at least 1 kHz) may be below 1 MHz, below 900 kHz, below 500 kHz, below 200 kHz, below 100 kHz, below 50 kHz or below 10 kHz. Further, the strength of time-varying magnetic field (the magnetic flux density) may be at least 0.1 T, at least 1 T, at least 2 T, at least 3 T, or at least 5 T. For example, the strength of time-varying magnetic field is between 2 and 3 T.

The modulation imposed on the time-varying magnetic field generated by at least one of the two magnetic field generating devices may be a phase or frequency modulation. If the time-varying magnetic field is phase (frequency) modulated, the phase (frequency) of the magnetic field varies over time (e.g. periodically). However, the invention is not restricted to a particular modulation scheme such that e.g. also an amplitude modulation scheme might be used. According to another embodiment of the invention, the carrier frequencies of the time-varying magnetic fields generated by the magnetic field generating devices are identical. It is noted that the term "carrier frequency" relates to the frequency of the (unmodulated) time-varying magnetic field (the "carrier magnetic field"). The carrier magnetic field may be continuous over time (e.g. non-pulsed), i.e. the time-varying magnetic field varies continuously (e.g. sinusoidally). The carrier magnetic field is modulated, e.g. phase or frequency modulated, wherein at least one of the time-varying magnetic fields is phase modulated. In case the magnetic and/or the induced electric field is not modulated (which may be the case for one of the magnetic fields), the carrier frequency is the (only) frequency of the time varying field.

The controlling arrangement may be configured to control the at least two magnetic field generating devices in such a way that the time-varying magnetic field generated by one of the magnetic field generating devices is phase modulated, while the time-varying magnetic field generated by the other magnetic field generating device is unmodulated.

Furthermore, the controlling arrangement may be configured to adjust the ratio of the amplitudes of the time-varying magnetic fields generated by the two magnetic field generating devices. The extent of amplitude modulation in particular depends on the vector sum of the two magnetic and/or induced electric fields at each location in the tissue. The modulation may be maximal when the two fields have equal magnitudes, and minimal near each magnetic field generating device, where one of the two fields may dominate the other.

The locus of maximum amplitude modulation and thus the locus of maximum stimulation may be steered by adjusting the relative amplitudes (i.e. the amplitude ratio) of the generated time-varying magnetic fields.

The modulation in particular is generated by applying a time-varying modulation function to the carrier magnetic field, wherein the modulation function, in principle, may be chosen arbitrarily to create an amplitude modulated resulting field whose envelope may be designed e.g. dependent on the desired effect on the tissue. The invention thus allows a high flexibility in the generation of stimulation waveforms. For example, the modulation (the modulation function) may be simply sinusoidal. However, more temporally complex resulting fields may be created. For example, short stimulation "pulses" may be emulated. For this, the modulation may be a phase modulation comprising a switch from a first state in which the time-varying magnetic fields have opposite phases to a second state in which the time-varying magnetic fields are in phase. At the location where the two magnetic and/or induced electric fields have equal magnitudes, the two fields will thus normally cancel out, and only transiently add up when they are brought in-phase, resulting in a stimulation pulse.

According to an embodiment of the invention, at least one of the magnetic field generating devices comprises or consists of at least one electromagnetic coil. However, the invention is of course not restricted to a particular embodiment of the two magnetic field generating devices. For example, at least one of the magnetic field generating devices may comprise or consists of at least one rotatable permanent magnet to generate the time-varying magnetic field.

In case the magnetic field generating devices are realized by means of electromagnetic coils, each magnetic field generating device may comprise at least one capacitor that combines with the coil to form a resonant circuit. The capacitance may be chosen so that the resonant frequency of the resonant circuit matches the required stimulation carrier frequency. The resulting circuit may have very low impedance at the resonant frequency and may be easily driven with low-voltage electronics. Other embodiments that do away with the capacitor and drive the coil directly using high-voltage electronics are also conceivable.

To overcome potential overheating issues of the stimulation coils, the coils may be actively cooled by a system that circulates water, oil or cooling liquid around them. If necessary, the stimulation could be performed in an intermittent manner, where the coils are turned off when no stimulation is required, and only turned on around the time points when stimulation pulses are required. However, sudden onsets and offset of electric fields are known to affect neural firing. To prevent this, currents may be slowly ramped up and down to and from their maximum values over a small time period (tens to hundreds of milliseconds) at the onset and offset of a stimulation pulse.

According to another embodiment of the invention, the two magnetic field generating devices belong to a first group and the system comprises a second group of magnetic field generating devices that contains at least two magnetic field generating devices for generating time-varying magnetic fields, wherein the controlling arrangement is configured to control at least one of the two magnetic field generating devices of the second group in such a way that the time-varying magnetic field generated by the device is modulated. Of course, more than two groups of at least two magnetic field generating devices each may be provided.

The magnetic fields generated by the devices of the first group may have the same carrier frequency and the magnetic fields generated by the magnetic field generating devices of the second group may have the same carrier frequency, wherein, however, the carrier frequency of the first group may be different from the carrier frequency of the second group. In order to e.g. ensure that low frequency amplitude modulation occurs only at the location where the fields from all the magnetic field generating devices are essentially equal, i.e. to prevent "extra" low frequency amplitude-modulated spots caused by interference of fields originating from magnetic field generating devices of different (two or more) groups, each group may operate at a different carrier frequency. The carrier frequencies are e.g. chosen so that the difference between any two of them is larger than a frequency that a neural tissue can respond to (e.g. at least 0.2 kHz, at least 0.5 kHz or at least 1 kHz). Then, at the location where all the fields add up equally, the envelope of the combined field will consist of a low frequency component at the modulation frequency, and some higher frequency components due to the beating between the different carrier frequencies. Since e.g. neural tissue only responds to low frequencies, it will react to the component at the modulation frequency and not to the higher frequency components.

The controlling arrangement of the system according to the invention may comprise a programmable unit, e.g. a microcontroller. The programmable unit may generate at least one control signal (driving signal) for controlling the time-varying magnetic fields generated by the two magnetic field generating devices. For example, the controlling arrangement comprises an amplifier circuit that receives the control signal and supplies the amplified control signal to the magnetic field generating devices. The control signal may determine both the time-varying magnetic field generated by the magnetic field generating device, i.e. the carrier magnetic signal, and the modulation of the time-varying magnetic field. In particular, the control signals set the waveform (including the frequency, amplitude and/or phase) of the modulated or unmodulated time-varying magnetic field. The corresponding control signal instead of being provided by the programmable unit may also be generated by analogue circuitry.

In case that the magnetic field generating devices each comprise an electromagnetic coil, the control signals generated by the programmable unit may determine the current supplied to the coils. In this embodiment, a Hall effect current sensor may be used to monitor the current in each coil (e.g. in real-time). Other types of current sensors may also be utilized. The current may be read by the programmable unit (e.g. the microcontroller mentioned above) which may implement a controller (e.g. a PID controller) that sets the frequency, amplitude and/or phase of the control signal for each coil in order to maintain the current frequency, amplitude and/or phase at the desired values and counteract any changes, e.g. due to temperature rises, phase modulation and/or inductive coupling between the coils. The controller could alternatively be implemented using analog electronics. The programmable unit may also monitor a coil temperature (e.g. in real-time) using a temperature sensor placed e.g. at the surface of each coil to ensure that the coil temperature remains within an admissible range. It is noted that the controlling arrangement of the system according to the invention does not necessarily have to realize a feedback loop, i.e. it does not have to be configured as a controller (such as the PID controller mentioned above), but only for steering the magnetic field generating devices for generating the time-varying magnetic fields.

According to a further embodiment of the invention, the system is battery-powered, wherein DC-DC converters (e.g. buck converters) may convert the battery voltage to a suitable level for powering components of the system such as the magnetic field generating devices for generating the time-varying magnetic fields and/or the programmable unit mentioned above. Furthermore, communication with external devices (e.g. electroencephalography amplifiers) may be supported through optically isolated digital I/O interfaces and/or Bluetooth. This may completely isolate the system from a power grid and external devices to reduce noise and ensure subject safety. Moreover, an over-current protection fuse and/or an emergency disconnect button may be integrated for safety in case of any failure in the system.

The invention also relates to a computer program product comprising program code having instructions which - when executed by a programmable unit of the controlling arrangement of the system of any of the preceding claims - will cause the controlling arrangement to control the two magnetic field generating devices of the system in such a way that each of them generates a time-varying magnetic field, wherein the time-varying magnetic field of at least one of the devices is modulated.

Embodiments of the invention will be described hereinafter with reference to the drawings, which show:
- Figure 1A: the strength of two electric fields induced by two time-varying magnetic fields generated by a system according to the invention, wherein one of the magnetic fields is phase modulated by means of a sinusoidal modulation function and has essentially the same amplitude as the unmodulated magnetic field;
- Figure 1B: a combined electric field resulting from interference of the electric fields depicted in Figure 1A;
- Figure 2A: the strength of two electric fields induced by two time-varying magnetic fields generated by a system according to the invention, wherein one of the magnetic fields is phase modulated and has a smaller amplitude than the unmodulated magnetic field;
- Figure 2B: a combined electric field resulting from interference of the electric fields depicted in Figure 2A;
- Figure 3A: the strength of two electric fields induced by two time-varying magnetic fields generated by a system according to the invention, wherein one of the magnetic fields is phase modulated by means of an arbitrary modulation function;
- Figure 3B: a combined electric field resulting from interference of the electric fields depicted in Figure 3A;
- Figure 4A: the strength of two electric fields induced by two time-varying magnetic fields generated by a system according to the invention, wherein one of the magnetic fields is phase modulated by means of an on-off-modulation function;
- Figure 4B: a combined electric field resulting from interference of the electric fields depicted in Figure 3A;
- Figures 5A-C: measurements of an electric field induced by the two time-varying magnetic fields generated by a system according to the invention;
- Figure 6: a block diagram of a system according to an embodiment of the invention; and
- Figure 7: schematically a perspective view of a system according to another embodiment of the invention arranged to stimulate tissue of the human brain.

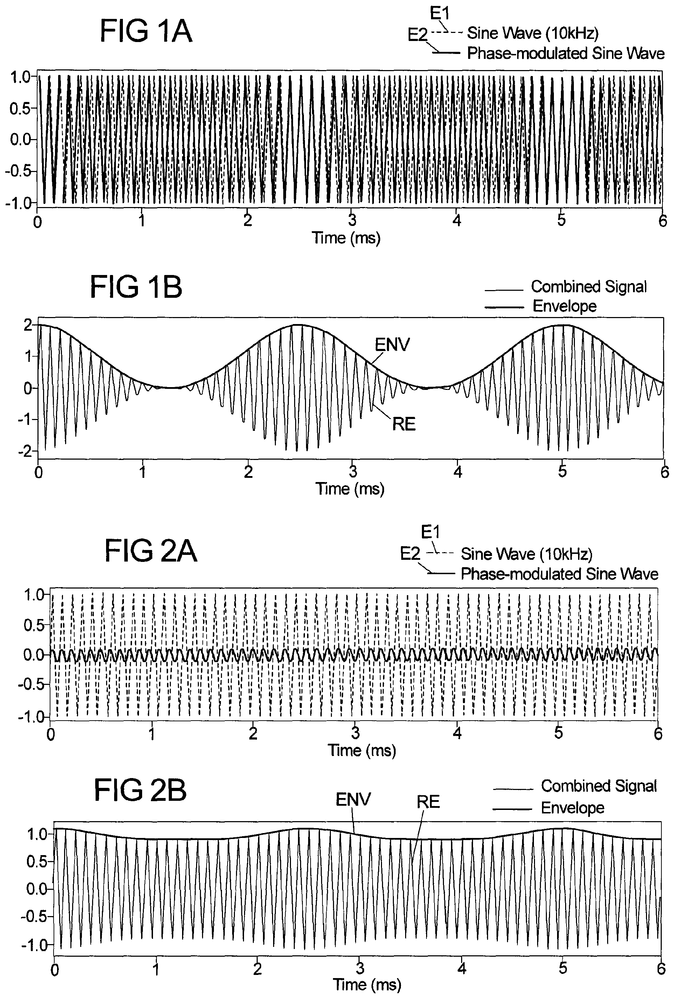

Figure 1 shows the (simulated) course of the strength of two electrical fields E1, E2, wherein the first electrical field E1 is induced in tissue of the human brain by a first time-varying magnetic field generated by a first magnetic field generating device of a system according to the invention. The second electrical field E2 is induced by a second time-varying magnetic field generated a second magnetic field generating device of the system.

The magnetic fields and thus the induced electrical fields E1, E2 are sinusoidal (having the same frequency), wherein the second magnetic field and thus the second electric field E2 is phase modulated, while the first magnetic field is unmodulated. The two magnetic field generating devices each may comprise an electromagnetic coil to which a time-varying current is supplied that creates the waveform of the emitted magnetic and thus of the induced electric fields.

The two coils are to be placed on the scalp. Each coil may consist of multiple windings of copper wire with either an air core or a ferromagnetic core. The two coils may be manufactured in such a way to be as similar as possible. The coils can be made of litz wire to minimize skin and proximity effects. Each coil has been driven with a high-frequency (e.g. in the kHz range) sinusoidal current (having e.g. several to tens of amps). The two coils have been driven at the same frequency, but the phase of the current in the one of coils that causes the second electric field E2 is modulated at a low frequency (e.g. 100 Hz). When the two induced electric fields E1, E2 combine in a region (overlap region) of the brain, the resulting field is amplitude-modulated at the phase modulation frequency. Figure 1A shows the strengths of the electric fields in the overlap region. The resulting electric field RE created by interference of the first and second electrical field E1, E2 is shown in Fig. 1A. RE has a carrier frequency identical to the carrier frequencies of the first and second electrical field E1, E2 and an envelope ENV having the frequency of the phase modulation. Of course, the first magnetic field could be modulated instead of the second magnetic field. Also, both magnetic fields may be modulated.

The extent of amplitude-modulation depends on the vector sum of the two electric fields E1, E2 at each location in the brain. The modulation is maximal when the two fields have equal magnitudes, and minimal near each coil, where one field dominates the other. As already set forth above, since neural tissue does not respond to unmodulated high-frequency electric fields, but responds to amplitude-modulated fields, neural modulation would be constrained to regions where the combined electric field exhibits large amplitude modulation. The locus of maximal amplitude-modulation can be steered by adjusting the relative magnitudes of the coil currents. Figures 1A and 1B relate to the situation that the amplitudes of the electric fields E1, E2 in the overlap region are essentially the same such that the amplitude of the resulting field RE becomes maximal.

Figures 2A and 2B, however, relate to the situation that the amplitudes of the electrical fields E1, E2 in the overlap region considered in Figures 1A and 1B are different. More particularly, the amplitude of the modulated second magnetic field and thus of the induced second electric field E2 is considerably smaller than the amplitude of the first magnetic field and thus of the first induced electric field E1. Accordingly, the amplitude of the resulting field RE is small.

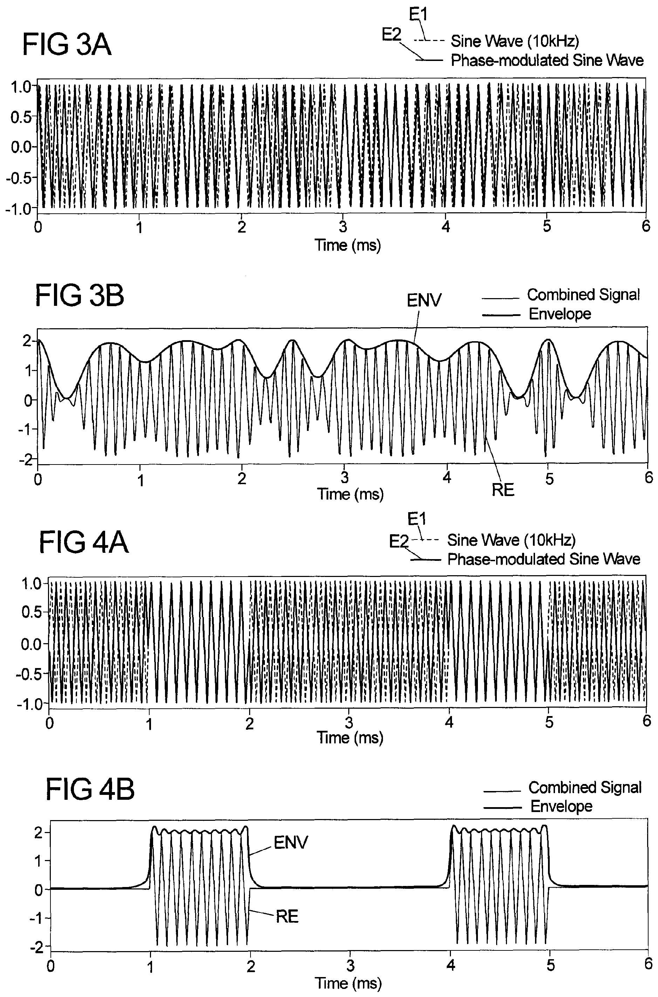

The phase modulation does not have be sinusoidal. Rather, in principle, any modulation waveform could be imposed on the magnetic field. Figures 3A, 3B are related to the embodiment that the second magnetic field and thus the second electric field E2 is arbitrarily modulated so that the combined (resulting) field RE has an arbitrary envelope ENV, allowing for high flexibility in creating stimulation waveforms.

Further, short stimulation pulses can be emulated by having the two magnetic and electric fields be normally anti-phasic and briefly reversing the phase of one of the coils to bring the coils transiently in-phase. This embodiment is reflected by Figures 4A and 4B. At the location where the two fields overlap and have essentially equal magnitudes, the two magnetic and electric fields will normally cancel out (during the anti-phasic intervals). Transiently (during the in-phase intervals) they will add up resulting in a stimulation pulse (see envelope ENV of resulting field RE).

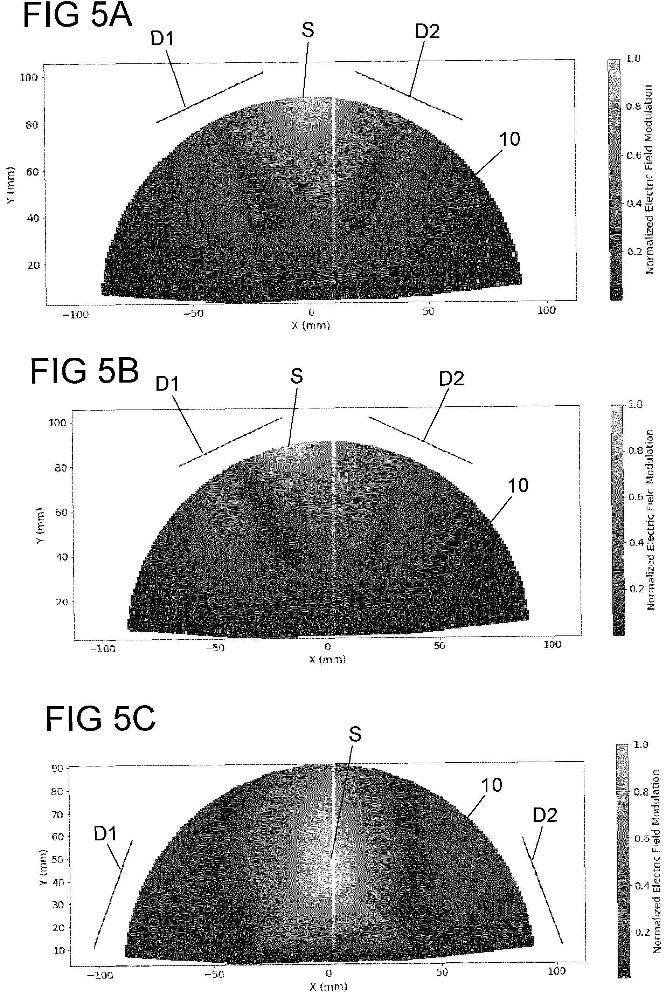

Figures 5A-5C show different arrangements of two magnetic field generating devices D1, D2 of a system according to the invention. According to Fig. 5A, the magnetic field generating devices D1, D2 are located in proximity to one another to target a cortical site at the midline of the coronal plane. The devices D1, D2 may comprise electromagnetic coils, wherein the coil currents are equal in both coils. Fig. 5A shows a measurement for that arrangement of the devices D1, D2, wherein the measurement shows the profile of an electric field induced by the magnetic fields emitted by devices D1, D2 in a spherical-conductor 10 approximation of the human brain. The induced electric field was probed at different locations on a coronal 2D plane by measuring the induced voltage in a triangular loop of copper wire. The gray scale indicates the extent of the amplitude modulation of the normalized electric field. As the currents supplied to the coils of the magnetic field generating devices D1, D2 and thus the magnetic and induced electric fields are essentially equal, the locus S of main stimulation appears on the midline.

According to Figure 5B, the location of the magnetic field generating devices D1, D2 has not been altered. However, the current in the coil of the first magnetic field generating devices D1 has been decreased relative to the second magnetic field generating devices D2. Correspondingly, the stimulation locus S moves towards the first (left) device D1.

In order to target a deep site of the brain, the magnetic field generating devices D1, D2 may be placed with a larger distance from the midline (Figure 5C). The point S of maximum stimulation for this configuration is then located deeper in the brain, e.g. approximately 45 mm deep.

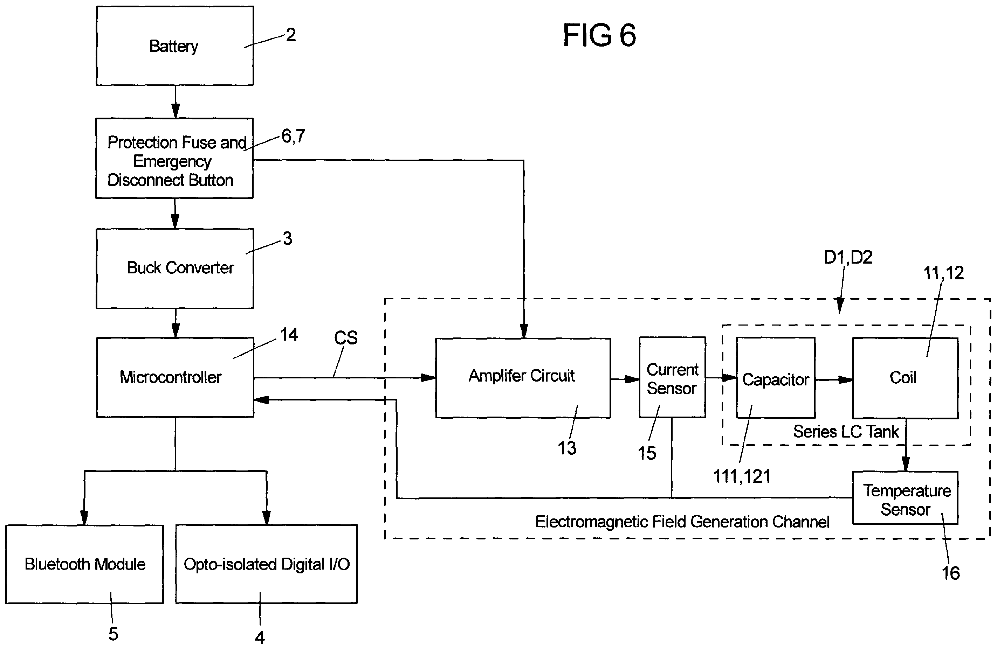

Figure 6 shows a block diagram of a system 100 according to the invention. The system 100 comprises a first and a second magnetic field generating device D1, D2, each having at least one electromagnetic coil 11, 12. Each coil 11, 12 is combined with a capacitor (e.g. a film capacitor) 111, 121 to form a resonant circuit RC1, RC2 (series LC tank). The capacitance of the capacitors 111, 121 is chosen so that the resonant frequency of the LC tank RC1. RC2 matches the desired stimulation carrier frequency of the magnetic fields to be generated. The resulting circuits RC1, RC2 have very low impedance at the resonant frequency and can be easily driven with low-voltage electronics. The capacitors 111, 121 could be omitted and the coils 11, 12 directly driven using high voltage.

The embodiment illustrated in Figure 6 utilizes a class-D amplifier circuit 13 to drive each LC tank RC1, RC2 with a sinusoidal voltage at the stimulation carrier frequency. The sine wave signal is generated using a digital-to-analog converter driven by a programmable unit in the form of a microcontroller 14. The LC tank RC1, RC2 could be alternatively be driven using an H-Bridge circuit that generates a square wave voltage at the stimulation carrier frequency with the appropriate phase. Further, more than one amplifier circuit 13 may be provided, wherein each one of the coils 11, 12 may be operated by their own amplifier circuit. The amplitude of the square wave could then be controlled by a DC-DC step-down buck converter. The resonant nature of the LC tank would filter the square wave voltage into a sinusoidal current at the fundamental frequency. Both implementations are highly efficient in terms of energy consumption due to the use of a high efficiency switching electronics, but other implementations that use analog electronics (e.g. class-AB amplifiers) are also possible. The amplifier circuit 13 and the microcontroller 14 form part of a controlling arrangement 50 of the system 100 for controlling the magnetic field generating devices D1, D2 (in particular the coils 11, 12). Using the controlling arrangement 50 the current supplied to the first and/or the second coil 11, 12 is modulated, e.g. phase modulated. In particular, a control signal CS generated by the microcontroller 14 steers the coil currents and invokes the modulation of the current of the one of the coil that produces the modulated field.

The coils 11, 12 can be actively cooled by a system that circulates water or oil around them. If necessary, the stimulation could be performed in an intermittent manner, where the coils are turned off when no stimulation is required, and only turned on around the time points when stimulation pulses are required. For example, as already mentioned above, the currents supplied to the coils may be slowly ramped up and down to and from their maximum values over a small time period (e.g. tens to hundreds of milliseconds) at the onset and offset of a stimulation pulse.

Further, a current sensor 15 (e.g. a Hall effect sensor) is used to monitor the current in each coil 11, 12 in real-time. The measured current value is transmitted to the microcontroller 14. The microcontroller 14 implements a PID controller that sets the amplitude and phase of a control signal transmitted from the microcontroller 14 to the amplifier circuit 13 for each coil 11, 12 in order to maintain the amplitude and phase of the current supplied to the coils 11, 12 at the desired values and counteract any changes due to temperature rises, phase modulation (other than the desired phase modulation), and/or inductive coupling between the coils 11, 12.

As already mentioned above, the PID controller could alternatively be implemented using analog electronics. The microcontroller 14 also monitors coil temperature in real-time using a temperature sensor 16 placed at the surface of each coil 11, 12 to ensure that the coil temperature remains within an admissible range.

The system 100 is battery-powered by means of at least one battery 2, wherein a buck converter 3 converts the battery voltage to a suitable level for powering the microcontroller 14. Further, the system 100 comprises optically isolated digital I/O and Bluetooth interfaces 4, 5 for permitting communication with external devices (e.g. electroencephalography amplifiers). The system may thus be isolated from a power grid and external devices to reduce noise and ensure subject safety. Furthermore, an over-current protection fuse 6 and emergency disconnect button 7 may be provided to respond to a failure in the device.

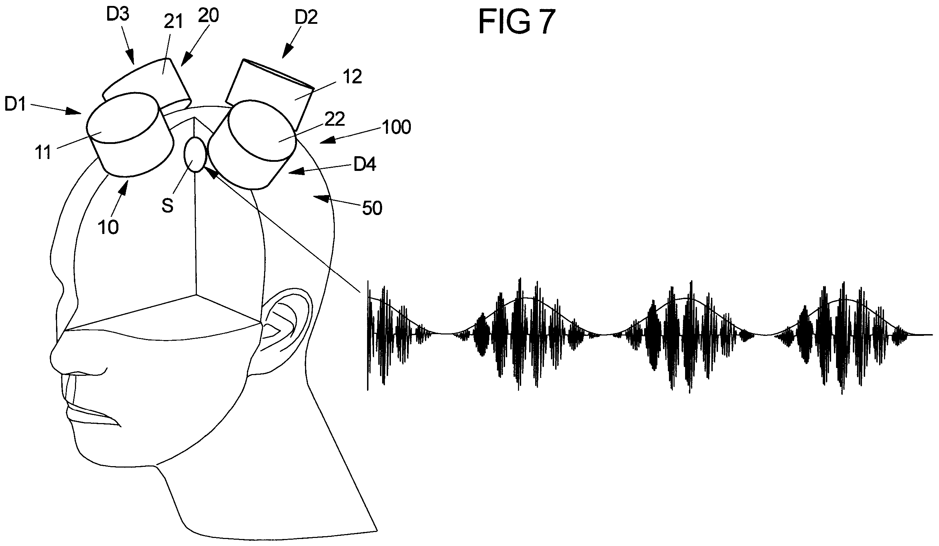

In order to increase stimulation strength and spatial resolution, the system 100 according to the invention may comprise more than two coils (or any other type of a magnetic field generating device) as shown in Figure 7. In this multi-coil embodiment, four magnetic field generating devices D1-D4 comprising coils 11, 12, 21, 22 are provided. The magnetic field generating devices D1-D4 and thus the coils 11, 12, 21, 22 are assigned to two groups, wherein the devices D1 and D2 (coils 11, 12) belong to a first group 10, while devices D3 and D4 (coils 21, 22) belong to a second group 20. The coils of each group 10, 20 are operated as described above e.g. in conjunction with Figure 6, in particular using a controlling arrangement 50. The magnetic field produced by of at least one of the magnetic field generating devices D1-D4 thus will be modulated; e.g. devices D2 (coil 12) and D4 (coil 22) produce a modulated signal. A common amplification circuit 13 may be used to operate the coils of each group 10, 20. However, it is also possible that at least one amplification circuit is assigned to each one of the groups 10, 20.

As already discussed above, in order to ensure that low frequency amplitude modulation occurs only at the location where the fields from all the coils 11, 12, 21, 22 are equal and to prevent undesired low frequency amplitude-modulated region between the coils of different groups 10, 20, the coils 11, 12, 21, 22 of each group 10, 20 operate at different carrier frequencies, i.e. the coils 11, 12 of the first group operate at a first carrier frequency and the coils 21, 22 of the second group 20 operate at a second carrier frequency different from the first carrier frequency. Therefore, the overlap region is the region S of maximum stimulation. The carrier frequencies of the groups 10, 20 are chosen in such a way that the difference between them is much larger than a frequency that a neural tissue can respond to (e.g. larger than 1 kHz). Accordingly, at a location where the fields generated and/or induced by the coils 11, 12, 21, 22 interfere, the envelope of the resulting field will have a low frequency component at the modulation frequency and some higher frequency components due to the different carrier frequencies of the two groups 10, 20. Of course, more than two groups may be used. Also, at least one of the groups may comprise more than two coils.

## Claims

1. A system for stimulation of biological tissue, comprising
- at least two magnetic field generating devices (D1-D4) for generating time-varying magnetic fields having carrier frequencies, wherein the magnetic field generating devices (D1-D4) are to be arranged relative to the tissue in such a way that their time-varying magnetic fields at least partially overlap in the tissue in an overlap region; and
- at least one controlling arrangement (50) for controlling the at least two magnetic field generating devices (D1-D4),
**characterised in that**
- the controlling arrangement (50) is configured to control at least one (D2, D4) of the at least two magnetic field generating devices (D1-D4) in such a way that the time-varying magnetic field generated by the at least one device (D2, D4) is phase modulated at a modulation frequency,
and
the controlling arrangement (50) is configured to control the at least one (D2, D4) of the at least two magnetic field generating devices (D1-D4) in such a way that a magnetic and/or an induced electric field resulting from an interference of the time-varying magnetic fields in the overlap region is amplitude-modulated at a frequency that depends on the modulation frequency.

2. The system of claim 1, wherein the controlling arrangement (50) is configured to control the at least one magnetic field generating device (D2, D4) in such a way that the time-varying magnetic field generated by the device (D2, D4) is frequency modulated.

3. The system of claim 1 or 2, wherein the controlling arrangement (50) is configured to control the at least two magnetic field generating devices (D1-D4) in such a way that the carrier frequencies of at least two of the time-varying magnetic fields generated by the devices (D1-D4) are identical.

4. The system of any of the preceding claims, wherein the controlling arrangement (50) is configured to adjust the ratio of the amplitudes of the time-varying magnetic fields generated by the at least two magnetic field generating devices (D1-D4).

5. The system of any of the preceding claims, wherein the frequency of the modulation is lower than a third or a fifth of the frequency of the time-varying magnetic field.

6. The system of any of the preceding claims, wherein the phase modulation comprises a switch from a first state in which the time-varying magnetic fields have opposite phases to a second state in which the time-varying magnetic fields are in phase.

7. The system of any of the preceding claims, wherein at least one of the magnetic field generating devices comprises or consist of at least one electromagnetic coil (11, 12, 21, 22).

8. The system of any of the preceding claims, wherein at least one of the magnetic field generating devices (D1-D4) comprises or consists of at least one rotatable permanent magnet.

9. The system of any of the preceding claims, wherein the at least two magnetic field generating devices (D1, D2) belong to a first group (10) of devices and the system (100) comprises a second group (20) of devices that contains at least two magnetic field generating devices (D3, D4) for generating time-varying magnetic fields, wherein the controlling arrangement (50) is configured to control at least one (D4) of the two magnetic field generating devices (D3, D4) of the second group (20) in such a way that the time-varying magnetic field generated by the device (D4) is modulated.

10. The system of claim 9, wherein the magnetic fields generated by the magnetic field generating devices (D1, D2) of the first group (10) have the same carrier frequency and the magnetic fields generated by the magnetic field generating devices (D3, D4) of the second group (20) have the same carrier frequency, and wherein the carrier frequency of the first group (10) is different from the carrier frequency of the second group (20).

11. The system of claim 10, wherein the difference between the carrier frequencies of the first and the second group (10, 20) is at least 0.2 kHz, at least 0.5 kHz or at least 1 kHz.

12. The system of any of the preceding claims, wherein the carrier frequency of at least one of the time-varying magnetic fields is at least 1 kHz.

13. The system of any of the preceding claims, wherein the carrier frequency of at least one of the time-varying magnetic fields is below 1 MHz, below 900 kHz, below 500 kHz, below 200 kHz, below 100 kHz, below 50 kHz or below 10 kHz.

14. The system of any of the preceding claims, wherein the controlling arrangement (50) is configured to control the at least two magnetic field generating devices (D1-D4) in such a way that the time-varying magnetic field generated by one of the magnetic field generating devices (D2, D4) is phase modulated, while the time-varying magnetic field generated by the other magnetic field generating device (D2, D4) is unmodulated.

15. A computer program product comprising program code having instructions which - when executed by a programmable unit (14) of the controlling arrangement (50) of the system (100) of any of the preceding claims - will cause the controlling arrangement (50) to control the at least two magnetic field generating devices (50) of the system (100) in such a way that each of them generates a time-varying magnetic field, wherein the time-varying magnetic field of at least one (D2, D4) of the devices (D1-D4) is phase modulated at a modulation frequency,
and wherein a magnetic and/or an induced electric field resulting from an interference of the time-varying magnetic fields in the overlap region is amplitude-modulated at a frequency that depends on the modulation frequency.

## Patentansprüche

1. System zur Stimulation von biologischem Gewebe, umfassend
- mindestens zwei Magnetfelderzeugungsvorrichtungen (D1-D4) zum Erzeugen von zeitlich variierenden Magnetfeldern mit Trägerfrequenzen, wobei die Magnetfelderzeugungsvorrichtungen (D1-D4) in einer solchen Weise relativ zu dem Gewebe anzuordnen sind, dass sich ihre zeitlich variierenden Magnetfelder wenigstens teilweise in dem Gewebe in einem Überlappungsbereich überlappen; und
- mindestens eine Steueranordnung (50) zum Steuern der mindestens zwei Magnetfelderzeugungsvorrichtungen (D1-D4),
**dadurch gekennzeichnet, dass**
- die Steueranordnung (50) dazu ausgebildet ist, wenigstens eine (D2, D4) der mindestens zwei Magnetfelderzeugungsvorrichtungen (D1-D4) in einer solchen Weise zu steuern, dass das von der mindestens einen Vorrichtung (D2, D4) erzeugte zeitlich variierende Magnetfeld mit einer Modulationsfrequenz phasenmoduliert wird, und
- die Steueranordnung (50) dazu ausgebildet ist, die mindestens eine (D2, D4) der mindestens zwei Magnetfelderzeugungsvorrichtungen (D1-D4) in einer solchen Weise zu steuern, dass ein aus einer Interferenz der zeitlich variierenden Magnetfelder resultierendes Magnetfeld und/oder ein induziertes elektrisches Feld in dem Überlappungsbereich mit einer Frequenz, die von der Modulationsfrequenz abhängt, amplitudenmoduliert wird.

2. System nach Anspruch 1, wobei die Steueranordnung (50) dazu ausgebildet ist, die mindestens eine Magnetfelderzeugungsvorrichtung (D2, D4) in einer solchen Weise zu steuern, dass das von der Vorrichtung (D2, D4) erzeugte zeitlich variierende Magnetfeld frequenzmoduliert wird.

3. System nach Anspruch 1 oder 2, wobei die Steueranordnung (50) dazu ausgebildet ist, die mindestens zwei Magnetfelderzeugungsvorrichtungen (D1-D4) in einer solchen Weise zu steuern, dass die Trägerfrequenzen von mindestens zwei der von den Vorrichtungen (D1-D4) erzeugten zeitlich variierenden Magnetfelder identisch sind.

4. System nach einem der vorhergehenden Ansprüche, wobei die Steueranordnung (50) dazu ausgebildet ist, das Verhältnis der Amplituden der von den mindestens zwei Magnetfelderzeugungsvorrichtungen (D1-D4) erzeugten zeitlich variierenden Magnetfelder einzustellen.

5. System nach einem der vorhergehenden Ansprüche, wobei die Frequenz der Modulation niedriger ist als ein Drittel oder ein Fünftel der Frequenz des zeitlich variierenden Magnetfelds.

6. System nach einem der vorhergehenden Ansprüche, wobei die Phasenmodulation ein Umschalten von einem ersten Zustand, in dem die zeitlich variierenden Magnetfelder entgegengesetzte Phasen haben, in einen zweiten Zustand, in dem die zeitlich variierenden Magnetfelder phasengleich sind, umfasst.

7. System nach einem der vorhergehenden Ansprüche, wobei mindestens eine der Magnetfelderzeugungsvorrichtungen mindestens eine elektromagnetische Spule (11, 12, 21, 22) umfasst oder daraus besteht.

8. System nach einem der vorhergehenden Ansprüche, wobei mindestens eine der Magnetfelderzeugungsvorrichtungen (D1-D4) mindestens einen drehbaren Permanentmagnet umfasst oder daraus besteht.

9. System nach einem der vorhergehenden Ansprüche, wobei die mindestens zwei Magnetfelderzeugungsvorrichtungen (D1, D2) zu einer ersten Gruppe (10) von Vorrichtungen gehören und das System (100) eine zweite Gruppe (20) von Vorrichtungen umfasst, die mindestens zwei Magnetfelderzeugungsvorrichtungen (D3, D4) zum Erzeugen von zeitlich variierenden Magnetfeldern enthält, wobei die Steueranordnung (50) dazu ausgebildet ist, mindestens eine (D4) der zwei Magnetfelderzeugungsvorrichtungen (D3, D4) der zweiten Gruppe (20) in einer solchen Weise zu steuern, dass das von der Vorrichtung (D4) erzeugte zeitlich variierende Magnetfeld moduliert wird.

10. System nach Anspruch 9, wobei die von den Magnetfelderzeugungsvorrichtungen (D1, D2) der ersten Gruppe (10) erzeugten Magnetfelder dieselbe Trägerfrequenz haben und die von den Magnetfelderzeugungsvorrichtungen (D3, D4) der zweiten Gruppe (20) erzeugten Magnetfelder dieselbe Trägerfrequenz haben, und wobei die Trägerfrequenz der ersten Gruppe (10) von der Trägerfrequenz der zweiten Gruppe (20) verschieden ist.

11. System nach Anspruch 10, wobei die Differenz zwischen den Trägerfrequenzen der ersten und der zweiten Gruppe (10, 20) mindestens 0,2 kHz, mindestens 0,5 kHz oder mindestens 1 kHz beträgt.

12. System nach einem der vorhergehenden Ansprüche, wobei die Trägerfrequenz von mindestens einem der zeitlich variierenden Magnetfelder mindestens 1 kHz beträgt.

13. System nach einem der vorhergehenden Ansprüche, wobei die Trägerfrequenz von mindestens einem der zeitlich variierenden Magnetfelder unter 1 MHz, unter 900 kHz, unter 500 kHz, unter 200 kHz, unter 100 kHz, unter 50 kHz oder unter 10 kHz liegt.

14. System nach einem der vorhergehenden Ansprüche, wobei die Steueranordnung (50) dazu ausgebildet ist, die mindestens zwei Magnetfelderzeugungsvorrichtungen (D1-D4) in einer solchen Weise zu steuern, dass das von einer der Magnetfelderzeugungsvorrichtungen (D2, D4) erzeugte zeitlich variierende Magnetfeld phasenmoduliert wird, während das von der anderen Magnetfelderzeugungsvorrichtung (D2, D4) erzeugte zeitlich variierende Magnetfeld unmoduliert ist.

15. Computerprogrammprodukt, das einen Programmcode mit Befehlen umfasst, die - bei Ausführung durch eine programmierbare Einheit (14) der Steueranordnung (50) des Systems (100) nach einem der vorhergehenden Ansprüche - die Steueranordnung (50) dazu veranlassen werden, die mindestens zwei Magnetfelderzeugungsvorrichtungen (D1-D4) des Systems (100) in einer solchen Weise zu steuern, dass jede von ihnen ein zeitlich variierendes Magnetfeld erzeugt, wobei das zeitlich variierende Magnetfeld von mindestens einer (D2, D4) der Vorrichtungen (D1-D4) mit einer Modulationsfrequenz phasenmoduliert wird, und wobei ein aus einer Interferenz der zeitlich variierenden Magnetfelder resultierendes Magnetfeld und/oder ein induziertes elektrisches Feld in dem Überlappungsbereich mit einer Frequenz, die von der Modulationsfrequenz abhängt, amplitudenmoduliert wird.

## Revendications

1. Système de stimulation des tissus biologiques, comprenant
- au moins deux dispositifs de génération de champ magnétique (D1-D4) pour générer des champs magnétiques variables dans le temps ayant des fréquences porteuses, les dispositifs de génération de champ magnétique (D1-D4) étant agencés par rapport au tissu de telle sorte que leurs champs magnétiques variables dans le temps se chevauchent au moins partiellement dans le tissu dans une zone de chevauchement ; et
- au moins un agencement de commande (50) pour commander les au moins deux dispositifs de génération de champ magnétique (D1-D4),
**caractérisé en ce que**
- l'agencement de commande (50) est configuré pour commander au moins un (D2, D4) des au moins deux dispositifs de génération de champ magnétique (D1-D4) de telle sorte que le champ magnétique variable dans le temps généré par le au moins un dispositif (D2, D4) soit modulé en phase à une fréquence de modulation, et
- l'agencement de commande (50) est configuré pour commander l'au moins un (D2, D4) des au moins deux dispositifs de génération de champ magnétique (D1-D4) de telle sorte qu'un champ magnétique et/ou un champ électrique induit résultant d'une interférence des champs magnétiques variables dans le temps dans la zone de chevauchement soit modulé en amplitude à une fréquence qui dépend de la fréquence de modulation.

2. Système selon la revendication 1, dans lequel l'agencement de commande (50) est configuré pour commander l'au moins un dispositif de génération de champ magnétique (D2, D4) de telle sorte que le champ magnétique variable dans le temps généré par le dispositif (D2, D4) soit modulé en fréquence.

3. Système selon la revendication 1 ou 2, dans lequel l'agencement de commande (50) est configuré pour commander les au moins deux dispositifs de génération de champ magnétique (D1-D4) de telle sorte que les fréquences porteuses d'au moins deux des champs magnétiques variables dans le temps générés par les dispositifs (D1-D4) soient identiques.

4. Système selon l'une quelconque des revendications précédentes, dans lequel l'agencement de commande (50) est configuré pour ajuster le rapport des amplitudes des champs magnétiques variables dans le temps générés par les au moins deux dispositifs de génération de champ magnétique (D1-D4).

5. Système selon l'une quelconque des revendications précédentes, dans lequel la fréquence de la modulation est inférieure à un tiers ou à un cinquième de la fréquence du champ magnétique variable dans le temps.

6. Système selon l'une quelconque des revendications précédentes, dans lequel la modulation de phase comprend un passage d'un premier état dans lequel les champs magnétiques variables dans le temps ont des phases opposées à un deuxième état dans lequel les champs magnétiques variables dans le temps sont en phase.

7. Système selon l'une quelconque des revendications précédentes, dans lequel au moins un des dispositifs de génération de champ magnétique comprend ou consiste en au moins une bobine électromagnétique (11, 12, 21, 22).

8. Système selon l'une quelconque des revendications précédentes, dans lequel au moins un des dispositifs de génération de champ magnétique (D1-D4) comprend ou consiste en au moins un aimant permanent rotatif.

9. Système selon l'une quelconque des revendications précédentes, dans lequel les au moins deux dispositifs de génération de champ magnétique (D1, D2) appartiennent à un premier groupe (10) de dispositifs et le système (100) comprend un deuxième groupe (20) de dispositifs qui contient au moins deux dispositifs de génération de champ magnétique (D3, D4) pour générer des champs magnétiques variables dans le temps, dans lequel l'agencement de commande (50) est configuré pour commander au moins un (D4) des deux dispositifs de génération de champ magnétique (D3, D4) du deuxième groupe (20) de telle sorte que le champ magnétique variable dans le temps généré par le dispositif (D4) soit modulé.

10. Système selon la revendication 9, dans lequel les champs magnétiques générés par les dispositifs de génération de champ magnétique (D1, D2) du premier groupe (10) ont la même fréquence porteuse et les champs magnétiques générés par les dispositifs de génération de champ magnétique (D3, D4) du deuxième groupe (20) ont la même fréquence porteuse, et dans lequel la fréquence porteuse du premier groupe (10) est différente de la fréquence porteuse du deuxième groupe (20).

11. Système selon la revendication 10, dans lequel la différence entre les fréquences porteuses du premier et du deuxième groupe (10, 20) est d'au moins 0,2 kHz, d'au moins 0,5 kHz ou d'au moins 1 kHz.

12. Système selon l'une quelconque des revendications précédentes, dans lequel la fréquence porteuse d'au moins un des champs magnétiques variable dans le temps est d'au moins 1 kHz.

13. Système selon l'une quelconque des revendications précédentes, dans lequel la fréquence porteuse d'au moins un des champs magnétiques variable dans le temps est inférieure à 1 MHz, inférieure à 900 kHz, inférieure à 500 kHz, inférieure à 200 kHz, inférieure à 100 kHz, inférieure à 50 kHz ou inférieure à 10 kHz.

14. Système selon l'une quelconque des revendications précédentes, dans lequel l'agencement de commande (50) est configuré pour commander les au moins deux dispositifs de génération de champ magnétique (D1-D4) de telle sorte que le champ magnétique variable dans le temps généré par l'un des dispositifs de génération de champ magnétique (D2, D4) soit modulé en phase, tandis que le champ magnétique variable dans le temps généré par l'autre dispositif de génération de champ magnétique (D2, D4) n'est pas modulé.

15. Produit programme d'ordinateur comprenant un code programme comportant des instructions qui - lorsqu'elles sont exécutées par une unité programmable (14) de l'agencement de commande (50) du système (100) selon l'une quelconque des revendications précédentes - amèneront l'agencement de commande (50) à commander les au moins deux dispositifs de génération de champ magnétique (50) du système (100) de telle sorte que chacun d'entre eux génère un champ magnétique variable dans le temps, dans lequel le champ magnétique variable dans le temps d'au moins un (D2, D4) des dispositifs (D1-D4) est modulé en phase à une fréquence de modulation, et dans lequel un champ magnétique et/ou un champ électrique induit résultant d'une interférence des champs magnétiques variables dans le temps dans la zone de chevauchement est modulé en amplitude à une fréquence qui dépend de la fréquence de modulation.
